# EUROPÄISCHE PATENTSCHRIFT

(11) **EP 2 303 209 B1**
(45) Veröffentlichungstag und Bekanntmachung des Hinweises auf die Patenterteilung: **01.05.2013**
(21) Anmeldenummer: 09768992.1
(22) Anmeldetag: 24.06.2009
(51) Int. Cl.: A61F 13/06, A61F 5/01, A61F 5/30

(54) **KNIEBANDAGE**
KNEE BANDAGE
BANDAGE DU GENOU

(30) Priorität: 25.06.2008 DE 102008029825
(43) Veröffentlichungstag der Anmeldung: 06.04.2011
(73) Patentinhaber: medi GmbH & Co. KG, 95448 Bayreuth (DE)
(72) Erfinder: HERRESTHAL, Jens, 60599 Frankfurt am Main (DE)
(74) Vertreter: Blaumeier, Jörg
(86) Internationale Anmeldenummer: PCT/EP2009/004565
(87) Internationale Veröffentlichungsnummer: WO 2009/156143

(56) Entgegenhaltungen:
- EP-A- 1 093 779
- DE-U1- 8 115 670
- DE-U1-202004 001 936
- US-A- 3 934 583
- US-A- 3 945 046

## Beschreibung

Die Erfindung betrifft eine Kniebandage.

Kniebandagen (oder: Kniegelenkbandagen) sind in vielfältigen Ausführungsformen bekannt. In besonderen Ausführungen haben Kniebandagen eine Funktion der mechanischen Korrektur von Luxationen der Kniescheibe (Patella). Dabei wird durch mechanische Hilfsmittel wie Bügel oder Bänder ein seitlicher Druck auf die Patella von außen ausgeübt, um der Luxation der Patella entgegenzuwirken. Solche Kniegelenkbandagen sind beispielsweise aus DE 10 2004 04 793 A1 oder aus DE 38 38 576 A1 oder aus US 6,287,269 B1 bekannt.

Die anatomische Führung der Patella erfolgt zum einen passiv durch die Trochlea, zum anderen ist sie in der Bewegung stark von der Dynamik der verschiedenen Muskelkräfte des seitlichen breiten Oberschenkelmuskel (Musculus vastus lateralis) und des zur Mitte gerichteten breiten Oberschenkelmuskel (Musculus vastus medialis) abhängig. Mittig und seitlich zur Kniescheibe und zum Kniescheibenband verläuft ein Band, das Retinaculum patellae, welches sich in einen mittleren Anteil, dem Retinaculum patellae mediale, welches aus dem Musculus vastus medialis entsteht, und einen seitlichen Anteil, dem Retinaculum patellae laterale, welches sich aus dem Musculus vastus lateralis bildet oder an dem einige Muskelfasern des Musculus vastus lateralis ansetzen und das an der anderen Seite mit dem lateralen (seitlichen) Rand (Margo lateralis) der Patella als Ansatzfläche verbunden ist, aufteilt. Der untere Bereich des Musculus vastus lateralis heftet sich distal am Femur (Oberschenkelknochen) sowie am lateralen Rand der Patella an und zieht mittels des Lig. Patella über das Knie. Während des Musculus vastus lateralis nun die Patella lateral oder nach außen zieht, zieht der Musculus vastus medialis die Patella nach innen oder medial. Im gesamten möglichen Bewegungsumfang des Knies muss das patellofemurale System, also das System von Patella zum Femur, durch ein ausbalanciertes muskuläres Zusammenspiel im Gleichgewicht bleiben. Eine isolierte Kontraktion des Musculus vastus lateralis beispielsweise ergibt eine Subluxation der Patella nach lateral.

Gerät das patellofemurale System aufgrund einer muskulären Dysbalance aus dem Gleichgewicht, so entsteht insbesondere ein abnormaler Patella-Tilt (Kippung der Kniescheibe), der im Unterschied zur Luxation eine vermehrte Belastung der lateralen Facette der lateralen Patelle verursacht. Eine adaptierte Verkürzung des lateralen Retinaculum oder eine Retinaculumfehlbelastung führt ebenso zu einem erhöhten Risiko eines retropatellaren Knorpeldefekts. Solche dauerhaften Fehlbelastungen können also zu einer schnelleren Knorpelabnutzung führen und können beispielsweise hervorgerufen werden durch eine längere Ruhigstellung des Muskels in verkürzter Stellung, z. B. vollständig extendiertes Knie im Sitzen oder Liegen.

Die Belastung von verkürzten Muskeln führt zudem zur Ausbildung von muskulären Triggerpunkten die lokale und fortgeleitete Schmerzen erzeugen. Die Triggerpunkte im distalen Musculus vastus lateralis, die zu einer Patellablockade führen können, liegen für gewöhnlich oberflächlich. Bei extendiertem Knie sind diese am besten aufzufinden.

Es ist eine ischämische Kompression (Beispiel: Tennisballmethode) bekannt, die eine Deaktivierung der meisten oberflächlichen Triggerpunkte des Musculus vastus lateralis bewirkt.

Aus US-A-3945046 ist eine Kniebandage bekannt, umfassend ein schlauch- oder röhrenförmiges Grundelement mit einem Kniescheibenbereich, einem Oberschenkelbereich und einem Unterschenkelbereich. Ferner sind Filzeinlagen als großflächige, lappenförmige Einlagen vorgesehen.

Der Erfindung liegt nun die Aufgabe zugrunde, eine neue Kniebandage anzugeben.

Diese Aufgabe wird gemäß der Erfindung gelöst mit den Merkmalen des Patentanspruchs 1. Weiterbildungen und Ausgestaltungen der Kniebandage gemäß der Erfindung ergeben sich aus den vom Patentanspruch 1 abhängigen Patentansprüchen.

Die Erfindung beruht auf der Erkenntnis, eine gezielte massierende Einwirkung einer an der Kniebandage vorgesehenen Massagepelotte (oder: Friktionspelotte) auf einen Triggerbereich oder Triggerpunkt des Musculus vastus lateralis vorzusehen. Es hat sich überraschenderweise herausgestellt, dass dadurch funktionelle Störungen oder Dysbalancen wie beispielsweise eine Verspannung oder Verkürzung des Musculus vastus lateralis gelindert oder positiv beeinflusst werden kann. Die Massagepelotte an der Kniebandage vermindert den Tonus des Musculus vastus lateralis, wodurch eine muskulär bestehende Dysbalance des Musculus vastus medialis gezielt verbessert und die gesamte Führung des patellofemuralen Systems optimiert werden kann. Insbesondere kann die Kniebandage gemäß der Erfindung durch eine Reduzierung des abnormalen Patellatilts und damit des retropatellaren Drucks Knorpelschäden und den möglichen Beginn einer Retropatellararthrose verhindern helfen oder auch helfen, einer Luxation oder Lateralisierung der Patella entgegenzuwirken. Diese neue Funktionsweise der Kniebandage kombiniert Kniegelenkstatik mit Muskelfunktion in synergistischer Weise.

Die Kniebandage gemäß der Erfindung umfasst deshalb an wenigstens einem Triggerbereich oder Triggerpunkt des Musculus vastus lateralis zur Kniescheibe hin wenigstens eine Massagepelotte, die mittels nach innen zum Bein hin gerichtete Massageelementen oder Massagepunkten oder -flächen an solchen Massageelementen, vor allem bei der Bewegung des Knies, fortlaufend auf diesen Triggerbereich oder -punkt am Musculus vastus lateralis unter einem Massagedruck und/oder Massagefriktion einwirkt und so den unerwünschten seitlich nach außen auf die Kniescheibe wirkenden Querkräften durch den Musculus vastus lateralis entgegenwirkt oder diese Querkräfte reduziert. Bevorzugt ist der Massagebereich, in dem die Massagepelotte angeordnet ist, dem Triggerbereich oder Triggerpunkt im distalen Randbereich oder Übergangsbereich des Musculus vastus lateralis, der zu dem oder in das Retinaculum laterale reicht, zugeordnet. Dieser Triggerpunkt wird auch als Triggerpunkt 1 des Musculus vastus lateralis bezeichnet.

Der Massagedruck der Massagepelotte wird zur Erzielung der gewünschten Wirkung definiert eingestellt. Um den Massagedruck durch die Massagepelotte diagnose- und patientenspezifisch einstellen zu können, ist wenigstens ein Spannelement zum Einstellen des Drucks der Massagepelotte nach innen auf den Triggerpunkt vorgesehen.

Das Spannelement umfasst insbesondere einen Spanngurt, der in seiner Länge verstellbar ist und/oder sich diagonal über den Oberschenkelbereich erstrecken kann.

Alternativ oder zusätzlich kann das Spannelement auch ein, insbesondere elastisches, Zwischenelement umfassen, das zwischen einen Spanngurt oder einen elastischen Bereich des Bandagengrundelements und den Massagebereich mit der Massagepelotte geklemmt oder angeordnet wird und dadurch einen zusätzlichen Druck auf den Massagebereich ausübt. Der Anpressdruck ist nun über die Dicke des Zwischenelements und auch dessen Elastizität einstellbar. Der Spanngurt kann somit als einfacher Gurt ohne Längenverstellung ausgebildet sein, insbesondere als klettfähiges Band.

Die Massagewirkung wird ferner durch die Gestaltung und das Material der Massagepelotte eingestellt oder beeinflusst.

So kann die Massagepelotte einen Grundkörper aufweisen und können die Massageelemente, insbesondere einstückig oder in einem gemeinsamen Formkörper, an dem Grundkörper ausgebildet sein, insbesondere in Form von, vorzugsweise halbschalen- oder halbkugelförmigen oder rippenförmigen oder wellenförmigen, Erhebungen. Die Massageelemente können bevorzugt Abmessungen senkrecht zum oder in einer Richtung weg vom Grundkörper in einem Bereich zwischen 1 mm und 8 mm aufweisen. Die Grundgestalt des Grundkörpers kann insbesondere rechteckig oder dreieckig oder trapezförmig oder oval sein.

Ein bevorzugtes Material für die Massagepelotte oder (zumindest) die Massageelemente ist ein Elastomer, insbesondere auf Silikonbasis oder Polysiloxanbasis, wobei das Elastomer vorzugsweise eine Shore A-Härte zwischen 20 und 80 Shore A, insbesondere zwischen 55 und 65 Shore A aufweist.

Zweckmäßig ist die Anordnung der Massagepelotte in einer, beispielsweise aufgenähten oder aufgeklebten, Tasche an der dem Bein zugewandten Innenseite des Bandagegrundelementes.

Weiterhin kann in einer weiteren Ausführungsform wenigstens ein Korrekturelement zur mechanischen Korrektur der Patellaluxation vorgesehen sein, insbesondere ein an der Außenseite der Patella angeordneter Korrekturgurt.

Es kann nun in einer Weiterbildung das Spannelement oder der Spanngurt wenigstens teilweise mit dem Korrekturelement oder Korrekturgurt integriert sein oder einstückig ausgebildet sein oder an diesem befestigt oder mit diesem verbunden sein.

Die Erfindung wird im Folgenden anhand von Ausführungsbeispielen weiter erläutert. Dabei wird auch auf die Zeichnungen Bezug genommen, in deren
- FIG 1: eine Kniebandage gemäß der Erfindung in einer perspektivischen Vorderansicht,

- FIG 2: die Kniebandage gemäß FIG 1 mit gelösten und teilweise nicht dargestellten Spanngurten sowie einer an der Innenseite befindlichen Massagepelotte in einer perspektivischen Ansicht und
- FIG 3: eine Massagepelotte für eine Kniebandage gemäß FIG 1 und FIG 2 in einer perspektivischen Ansicht
- FIG 4: eine weitere Ausführungsform einer Kniebandage gemäß der Erfindung in einer perspektivischen Ansicht und
jeweils schematisch dargestellt sind. Einander entsprechende Teile und Größen sind in FIG 1 bis 4 mit denselben Bezugszeichen versehen.

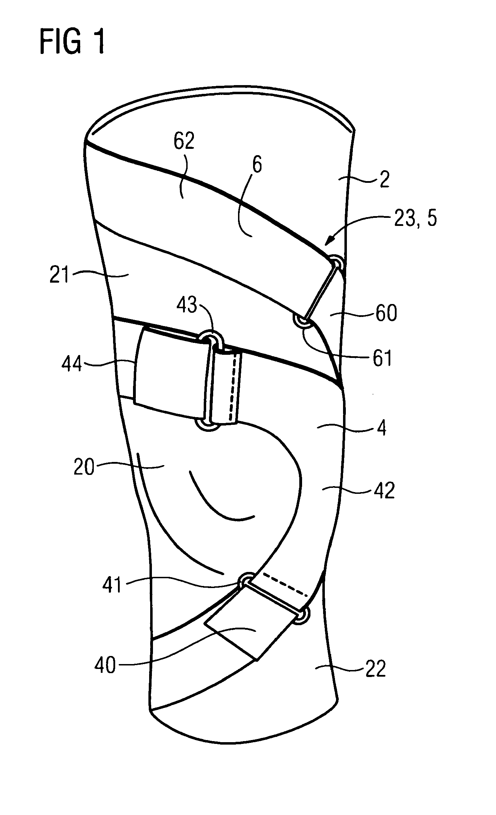

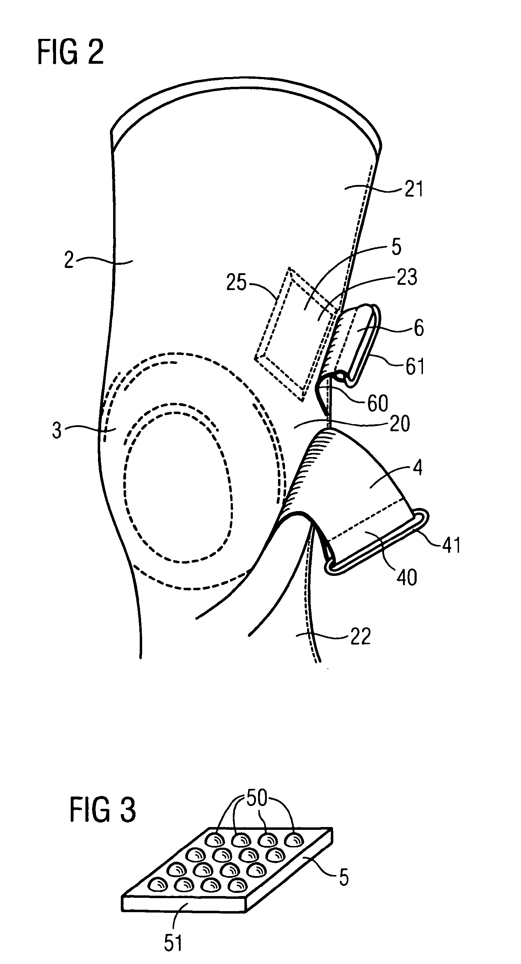

Die Kniebandage gemäß FIG 1 und 2 umfasst ein insbesondere schlauch- oder röhrenartiges Bandagengrundelement 2, das in Regel aus einem elastischen Material, insbesondere einem Textil, einem Gewebe oder einem Gewirke oder Gestrick, besteht. Geeignete Materialien sind, ohne Beschränkung der Allgemeinheit, textile Materialien auf Basis von Kunststofffasern oder auch Baumwolle.

Das Bandagengrundelement 2 umschließt im angezogenen Zustand vollständig das betroffene Knie sowie einen anschließenden Teilbereich des Unterschenkels und des Oberschenkels. Der zentrale, das Kniegelenk selbst, die Kniekehle und die Kniescheibe umschließende Kniescheibenbereich ist mit 20 bezeichnet, der zum Oberschenkel hin anschließende Oberschenkelbereich mit 21 und der zum Unterschenkel anschließende Unterschenkelbereich mit 22.

Im Kniescheibenbereich 20 ist an dem Bandagengrundelement 2 ein ringförmiges Randelement (oder Ringpelotte) 3 befestigt, das die nicht dargestellte Kniescheibe oder Patella umschließt und insbesondere aus einem elastischen und/oder dämpfenden Material, beispielsweise einem Silikonelastomer (Elastomer auf Polysiloxanbasis) oder einem anderen Elastomer, besteht. Das Randelement 3 ist an der dem Knie oder Bein zugewandten Innenfläche des Bandagengrundelements 2 direkt durch Kleben oder indirekt durch Einschließen in eine entsprechende Tasche befestigt.

An der Außenseite des Bandagengrundelements 2 ist das Randelement 3 von einem halbmond- oder annähernd halbkreisförmigen oder auch halbovalen Korrekturband (oder: Korrekturgurt) 42 umgeben, das in seinem zentralen Bereich an dem Bandagengrundelement 2 außen befestigt, insbesondere verklebt oder vernäht, ist. Das Korrekturband 42 ist an seinen freien Enden über jeweils eine von zwei Ösen (oder: langgestreckte Ringe) 41 und 43 jeweils mit einem ersten Spanngurt 40, der unterhalb des Randelements 3 verläuft und im Unterschenkelbereich 22 des Bandagengrundelements 2 befestigt ist, und mit einem zweiten Spanngurt 44, der oberhalb des Randelements 3 verläuft und insbesondere im Übergangsbereich zwischen dem Kniescheibenbereich 20 und dem Oberschenkelbereich 21 des Bandagengrundelements 2 befestigt ist, nach innen oder medial spannbar. Die beiden Spanngurte 40 und 44 können dazu über nicht dargestellte Klettelemente in verschiedenen Längen und damit unterschiedlich stark gespannt werden, so dass die von dem Korrekturband 42 auf die Kniescheibe von außen medial nach innen einwirkende Korrekturkraft auf die Kniescheibe zur mechanischen Korrektur einer Luxation eingestellt werden kann.

Oberhalb des Korrekturbandes 42 ist nun an dem Bandagengrundelement 2 an der dem Bein zugewandten Innenseite des Bandagengrundelements 2 in einem Massagebereich 23 eine Massagepelotte 5 an dem Bandagegrundelement 2 befestigt, insbesondere in einer Tasche 25 an der Innenseite angeordnet. Die Tasche 25 ist ringsum geschlossen und kann insbesondere mit einem an der Innenseite des Bandagengrundelements 2 verklebten oder vernähten Stück textilem Material gebildet sein.

Die Massagepelotte 5 kann, wie in FIG 3 dargestellt, einzelne Massageelemente 50 an einem Grundkörper 51 aufweisen, die insbesondere punktförmige Massageflächen oder Massagepunkte aufweisen, insbesondere durch Ausbildung in Form von Halbschalen oder halbkugelförmigen Elementen. Ebenso können aber auch in einer nicht dargestellten Ausführungsform auch Massageelemente mit linienartigen Massageflächen, insbesondere in Form von Rippen oder Wellen, an der Massagepelotte vorgesehen sein. Die Massageelemente 50 erstrecken sich von der Innenseite des Bandagengrundelements 2 weg auf das Bein zu und wirken durch das Material der Tasche 25 hindurch massierend auf den unter dem Massagebereich 23 des Bandagengrundelements 2 liegenden Bereich des Beins des Patienten. Die Massagepelotte 5 besteht vorzugsweise ebenfalls aus einem Silikonelastomer (Elastomer auf Polysiloxanbasis) oder einem anderen Elastomer. Die Masssagepelotte 5 weist vorzugsweise eine für den gewünschten Massageeffekt geeignet Elastizität auf, wobei vorzugsweise eine Shore A-Härte der Massagepelotte 5 zwischen 20 und 80 Shore A, insbesondere zwischen 55 und 65 Shore A, gewählt ist.

Der Grundkörper 51 der Massagepelotte 5 weist im Ausführungsbeispiel der FIG 3 eine längliche rechteckige Grundform auf und hat eine weitgehend konstante Dicke, zumindest außerhalb der Bereiche mit den Massageelementen 50.

Die Massagepelotte 5 oder deren Grundkörper 51 kann aber auch eine andere Gestalt aufweisen, beispielsweise eine ovale, polygonale, insbesondere dreieckige oder trapezförmige, Grundgestalt und zusätzlich oder alternativ auch etwas gekrümmt verlaufen in Anpassung an die Knie- und Beinform an der Stelle des Massagebereichs 23. Die Grundfläche des Grundkörpers 51 wird der Größe des Massagebereichs 23 angepasst. Die Form, Länge und Dicke der Massageelemente 50 und die Flächendichte ihrer Anordnung auf dem Grundkörper 51 sowie die Dicke des Grundkörpers 51 selbst werden dem gewünschten Massageeffekt angepasst. Typische Werte für die Dicke des Grundkörpers 51 sind 2 mm bis 8 mm und für die Länge der Massageelemente 50, vom Grundkörper 51 nach außen gemessen, 1 mm bis 8 mm.

Der Massagebereich 23 der Kniebandage bzw. dessen Bandagengrundelements 2 ist nun so angeordnet, dass er bei angezogener Kniebandage direkt an einem Triggerpunkt oder Triggerbereich des Musculus vastus lateralis zur Patella hin zu liegen kommt, vorzugsweise an oder über dem Triggerpunkt 1 des Musculus vastus lateralis, also anatomisch gesehen am distalen Randbereich oder Übergangsbereich des Musculus vastus laterlalis in das Retinaculum patellae laterale reichend.

Dadurch kann die im Massagebereich 23 angeordnete Massagepelotte 5 massierend auf den darunter liegenden Triggerpunkt- oder -bereich des Musculus vastus lateralis einwirken, einerseits senkrecht nach innen, andererseits auch durch die Bewegung in seitlicher oder entlang der Oberfläche. Es resultiert ein Massageeffekt durch die Massagepelotte 5 der Kniebandage, durch den Verspannungen und Verkürzungen des Triggerbereiches oder -punktes des Musculus vastus lateralis zur Patella hin gelindert werden und damit einer durch den Vastus lateralis ausgeübten nach außen oder lateral wirkenden Kraft auf die Kniescheibe entgegengewirkt wird. Insbesondere wird dadurch ein abnormaler, pathologischer Tilt (Verkippung) der Patella gemindert.

An oder in dem Massagebereich 23 ist nun ein Spanngurt 6 vorgesehen, der sich schräg über den Oberschenkelbereich 21 des Bandagegrundelements 2 bis hin zum Kniescheibenbereich 20 oder Übergangsbereich zum Kniescheibenbereich 20 erstreckt und zwei Spanngurtteile 60 und 62 aufweist, die an den voneinander abgewandten Endbereichen jeweils an dem Bandagengrundelement 2 an dessen vom Bein abgewandten Außenfläche befestigt, insbesondere verklebt oder vernäht, sind und an den einander zugewandten Bereichen oder Enden über eine Öse (oder: ein langgestrecktes Ringelement) 61 miteinander verbunden oder verbindbar sind. Dabei sind Klettelemente an dem längeren Spanngurtteil 62 vorgesehen, über die die Länge dieses Spanngurtteils 62 eingestellt werden kann und damit die Zugkraft oder Spannkraft des gesamten Spanngurtes 6 durch Veränderung der Länge des Spanngurtes eingestellt werden kann. Der Spanngurt 6 wirkt dadurch mit unterschiedlich einstellbarem Druck auf den unter dem Spanngurt 6, insbesondere unter dem ersten Spanngurtteil 60 und der Öse 61, befindlichen Massagebereich 23 und die dort befindliche Massagepelotte 5 abhängig von der eingestellten Länge des zweiten Spanngurtteils 62. Dadurch kann durch einen unterschiedlichen Spanndruck mittels des Spanngurtes 6 der Massagedruck, der durch die Massageelemente 50 an der Massagepelotte 5 nach innen auf den Triggerpunkt des Musculus vastus lateralis wirkt, gezielt eingestellt werden, je nach Patient und nach Diagnose.

Das kürzere Spanngurtteil 60 ist vorzugsweise fest mit der Öse 61 verbunden und in seiner Länge nicht verstellbar. Das längere Spanngurtteil 62 ist über Klettverschlüsse oder Klettelemente in seiner Länge verstellbar und erstreckt sich quer über den gesamten Oberschenkelbereich 21 zur entgegengesetzten (medialen) Seite, während das kürzere Spanngurtelement 60 an der distalen Seite des Bandagengrundelements 2 angeordnet ist, in dessen Oberschenkelbereich 21. Durch die diagonale Anordnung des Spanngurtes 6 erhält man einen große Längeneinstellbereich und damit eine gute und variable Spannwirkung und Ausübung eines Massagedrucks.

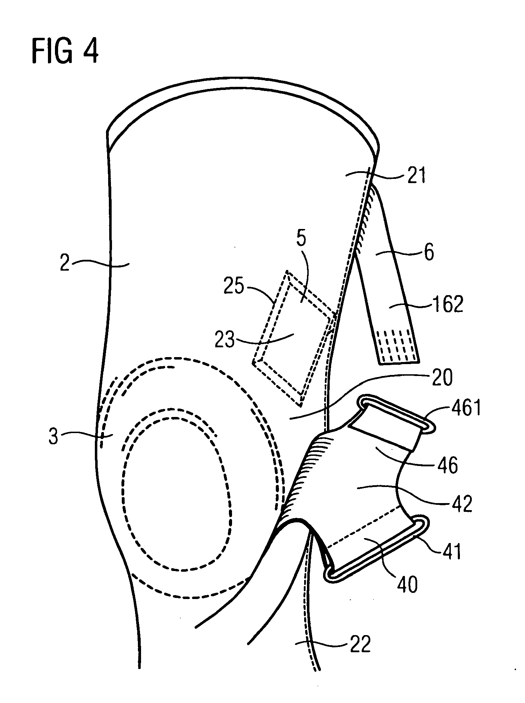

In dem Ausführungsbeispiel gemäß FIG 4 ist an dem Korrekturband oder Korrekturgurt 42 für das Randelement 3 der Kniescheibe ein Teil 46 des Spanngurtes 6 für den Massagebereich 23 und die Massagepelotte 5 ausgebildet. Das Korrekturband 42 ist dazu annähernd T-förmig ausgebildet mit einem Gurt 40 und einer Öse 41 ähnlich wie in FIG 2 und zusätzlich einem Spanngurtteil 46 mit einer Öse 461, an dem bzw. die ein zweiter Spanngurtteil 162 des Spanngurtes 6 befestigbar ist. Der im befestigten Zustand aus den beiden Spanngurtteilen 46 und 162 zusammengesetzte Spanngurt 6 verläuft nun quer oder diagonal von außen oder distal in medialer Richtung zur Kniescheibe oder dem Randelement 3 hin über den Massagebereich und durch Klettelemente am Spanngurtteil 162 kann wieder die Spannung oder Anpresskraft der Massagepelotte 5 eingestellt werden.

In diesem Ausführungsbeispiel ist also der Spanngurt teilweise am Korrekturgurt integriert oder einstückig ausgebildet. In einer Abwandlung könnte man auch den Korrekturgurt 42 an dem Bandagengrundelement 2 verschweißen oder vernähen und auch den Spanngurt 6 an dieser Schweiß- oder Nähnaht befestigen. Schließlich könnten auch Spanngurt und Korrekturgurt aus einem Teil gestanzt werden und am äußeren oberen Rand befestigt werden.

### Bezugszeichenliste

- 2: Bandagengrundelement
- 3: Randelement
- 5: Massagepelotte
- 6: Spanngurt
- 20: Kniescheibenbereich
- 21: Oberschenkelbereich
- 22: Unterschenkelbereich
- 23: Massagebereich
- 25: Tasche
- 41: Öse
- 42: Korrekturband
- 43: Öse
- 44: Spanngurt
- 46: Spanngurtteil
- 50: Massageelement
- 51: Grundkörper
- 60: Spanngurtteil
- 61: Öse
- 62: Spanngurtteil
- 162: Spanngurtteil
- 461: Öse

## Patentansprüche

1. Kniebandage umfassend
a) ein schlauch- oder röhrenförmiges Bandagengrundelement (2) mit einem der Kniescheibe zugeordneten zentralen Kniescheibenbereich (20), einem sich an den Kniescheibenbereich (20) anschließenden und einem Teilbereich des Oberschenkels zugeordneten Oberschenkelbereich (21) und einem sich an den Kniescheibenbereich (20) anschließenden und einem Teilbereich des Unterschenkels zugeordneten Unterschenkelbereich (22),
b) wenigstens eine Massagepelotte (5), die in einem Massagebereich (23) an dem Bandagengrundelement (2) befestigt ist und nach innen, bei angezogener Kniebandage zum Bein hin gerichtete Massageelemente (50) aufweist,
c) wobei der Massagebereich (23) mit der wenigstens einen Massagepelotte (5) im Oberschenkelbereich (21) angeordnet ist,
d) und wenigstens ein Spannelement (6) zum Einstellen des Drucks der Massagepelotte nach innen auf das Bein vorgesehen ist.

2. Kniebandage nach Anspruch 1, bei der die Massagepelotte (5) einen Grundkörper (51) aufweist und die Massageelemente (50) an dem Grundkörper (51) ausgebildet oder vorgesehen sind, insbesondere in Form von Erhebungen.

3. Kniebandage nach Anspruch 2, bei der wenigstens ein Teil der Massageelemente (50) an dem Grundkörper (51) in Form von halbschalen- oder halbkugelförmigen Erhebungen ausgebildet oder vorgesehen sind.

4. Kniebandage nach Anspruch 2 oder Anspruch 3, bei der wenigstens ein Teil der Massageelemente (50) an dem Grundkörper (51) in Form von rippenförmigen oder wellenförmigen Erhebungen ausgebildet oder vorgesehen sind.

5. Kniebandage nach einem der Ansprüche 2 bis 4, bei der die Massageelemente (50) Abmessungen senkrecht zum oder in einer Richtung weg vom Grundkörper (51) in einem Bereich zwischen 1 mm und 8 mm aufweisen.

6. Kniebandage nach einem der vorhergehenden Ansprüche, bei der die Massagepelotte (5) oder die Massageelemente (50) aus einem Elastomer gebildet ist, insbesondere einem Elastomer auf Silikonbasis oder Polysiloxanbasis, und vorzugsweise eine Shore A-Härte zwisehen 20 und 80 Shore A, insbesondere zwischen 55 und 65 Shore A aufweist.

7. Kniebandage nach einem der vorhergehenden Ansprüche, bei der die Massagepelotte (5) in einer Tasche (25) an der dem Bein zugewandten Innenseite des Bandagegrundelementes (2) angeordnet ist.

8. Kniebandage nach einem der vorhergehenden Ansprüche mit wenigstens einem Korrekturelement (42), insbesondere einem Korrekturband oder einem Korrekturbügel, das an dem Bandagengrundelement (2) an einer lateralen Seite der Kniescheibe angeordnet ist zum Ausüben einer seitlichen Kraft auf die Kniescheibe medial nach innen.

9. Kniebandage nach einem der vorangehenden Ansprüche, bei der das Spannelement einen Spanngurt (6) umfasst, der sich schräg über den Oberschenkelbereich (21) des Bandagegrundelements (2) bis hin zum Übergangsbereich zum Kniescheibenbereich (20) oder zum Kniescheibenbereich erstreckt und/oder der in seiner Länge zum Verändern der Spannkraft verstellbar ist.

10. Kniebandage nach Anspruch 9, bei der der Spanngurt (6) zwei Spanngurtteile (60, 62) aufweist, die an den voneinander abgewandten Endbereichen jeweils an dem Bandagengrundelement (2), insbesondere an dessen vom Bein abgewandter Außenfläche, befestigt, insbesondere verklebt oder vernäht, sind und an den einander zugewandten Bereichen oder Enden über ein Verbindungselement, insbesondere eine Öse (61), miteinander verbunden oder verbindbar sind.

11. Kniebandage nach Anspruch 9 oder Anspruch 10, bei der Klettelemente an einem der Spanngurtteile (62) vorgesehen sind, über die die Länge dieses Spanngurtteils (62) eingestellt werden kann.

12. Kniebandage nach einem der vorangehenden Ansprüche, bei der das Spannelement wenigstens ein, insbesondere elastisches, Zwischenelement umfasst, das zwischen einem Spanngurt oder Spannband oder einem elastischen Bereich des Bandagengrundelementes einerseits und dem Massagebereich mit der Massagepelotte andererseits geklemmt oder klemmbar oder angeordnet oder anordenbar ist und dadurch einen Druck auf den Massagebereich ausübt.

13. Kniebandage nach Anspruch 12, bei der der Anpressdruck über die Dicke des Zwischenelementes und ggf. dessen Elastizität einstellbar ist und/oder bei der wenigstens zwei Zwischenelemente umfasst sind, die gegeneinander austauschbar sind und unterschiedliche Dicken und/oder Elastizitäten aufweisen.

14. Kniebandage nach einem der vorhergehenden Ansprüche, bei der der Massagebereich mit der Massagepelotte dem Triggerpunkt oder -bereich im distalen Randbereich oder Übergangsbereich des Musculus vastus lateralis, der zu dem oder in das Retinaculum patellae laterale reicht, zugeordnet ist.

## Claims

1. A knee bandage comprising
a) a hose-shaped or tubular bandage base element (2) with a central patellar region (20) associated with the patella, a thigh region (21) adjacent to the patellar region (20) and associated with a partial region of the thigh, and a lower-leg region (22) adjacent to the patellar region (20) and associated with a partial region of the lower leg,
b) at least one massage pad (5), which is attached in a massage region (23) on the bandage base element (2) and provides massage elements (50) facing inwards towards the leg when the knee bandage is fitted,
c) wherein the massage region (23) with the at least one massage pad (5) is arranged in the thigh region (21),
d) and at least one tightening element (6) is provided to adjust the inward pressure of the massage pad on the leg.

2. The knee bandage according to claim 1, wherein the massage pad (5) provides a base body (51), and the massage elements (50) are embodied or provided on the base body (51), especially in the form of swellings.

3. The knee bandage according to claim 2, wherein at least some of the massage elements (50) are embodied or provided on the base body (51) in the form of half-cup or hemispherical swellings.

4. The knee bandage according to claim 2 or claim 3, wherein at least some of the massage elements (50) are embodied or provided on the base body (51) in the form of rib-shaped or corrugated swellings.

5. The knee bandage according to any one of claims 2 and 4, wherein the massage elements (50) provide dimensions within a range between 1 mm and 8 mm perpendicular to or in a direction facing away from the base body (51).

6. The knee bandage according to any one of the preceding claims, wherein the massage pad (5) or the massage elements (50) is formed from an elastomer, especially an elastomer based on silicon or polysiloxane, and preferably provides a Shore-A hardness between 20 and 80 Shore A, especially between 55 and 65 Shore A.

7. The knee bandage according to any one of the preceding claims, wherein the massage pad (5) is arranged in a pocket (25) facing towards the leg on the inside of the bandage base element (2).

8. The knee bandage according to any one of the preceding claims with at least one correction element (42), especially a correction tape or a correction bar, which is arranged on the bandage base element (2) on a lateral side of the patella in order to exert a lateral, medially inward force on the patella.

9. The knee bandage according to any one of the preceding claims, wherein the tightening element comprises a tightening strap (6), which extends diagonally over the thigh region (21) of the bandage base element (2) up to the transition region to the patellar region (20) or up to the patellar region and/or which is adjustable in its length in order to vary the tightening force.

10. The knee bandage according to claim 9, wherein the tightening strap (6) provides two tightening-strap components (60, 62), which are attached, especially glued or stitched on the end regions facing respectively away from one another on the bandage base element (2), especially on its outer surface facing away from the leg, and are connected or can be connected to one another on the regions or ends facing towards one another via a connecting element, especially an eyelet (61).

11. The knee bandage according to claim 9 or claim 10, wherein Velcro elements are provided on one of the tightening-strap components (62), by means of which the length of this tightening-strap component (62) can be adjusted.

12. The knee bandage according to any one of the preceding claims, wherein the tightening element comprises at least one, especially elastic, intermediate element, which is clamped or can be clamped, or is arranged or can be arranged between, on the one side, the tightening strap or tightening tape or an elastic region of the bandage base element and, on the other side, the massage region with the massage pad, and which therefore exerts a pressure on the massage region.

13. The knee bandage according to claim 12, wherein the pressure applied is adjustable via the thickness of the intermediate element and optionally its elasticity, and/or wherein at least two intermediate elements are provided, which are exchangeable with one another and comprise different thicknesses and/or elasticities.

14. The knee bandage according to any one of the preceding claims, wherein the massage region with the massage pad is associated with the trigger point or region in the distal edge region or transitional region of the musculus vastus lateralis which extends up to or into the retinaculum patellae.

## Revendications

1. Bandage pour genou comprenant :
a) un élément de base de bandage en forme de tuyau ou de tube (2) avec une zone centrale pour rotule (20) affectée à la rotule, une zone pour cuisse (21) adjacente à la zone pour rotule (20) et affectée à une zone partielle de la cuisse et une zone pour jambe (22) adjacente à la zone pour rotule (20) et affectée à une zone partielle de la jambe ;
b) au moins une pelote de massage (5) qui est fixée dans une zone de massage (23) sur l'élément de base du bandage (2) et présente vers l'intérieur, lorsque le bandage pour genou est porté, des éléments de massage (50) positionnés en direction de la jambe ;
c) étant entendu que la zone de massage (23) comprenant l'au moins une pelote de massage (5) est agencée dans la zone pour cuisse (21) ;
d) et il est prévu au moins un élément de serrage (6) destiné à régler la pression de la pelote de massage vers l'intérieur sur la jambe.

2. Bandage pour genou selon la revendication 1, dans lequel la pelote de massage (5) présente un corps de base (51) et les éléments de massage (50) sont réalisés ou prévus sur le corps de base (51), en particulier sous la forme d'élévations.

3. Bandage pour genou selon la revendication 2, dans lequel au moins une partie des éléments de massage (50) sont réalisés ou prévus sur le corps de base (51) sous la forme d'élévations en forme de demi-coques ou de demi-sphères.

4. Bandage pour genou selon la revendication 2 ou la revendication 3, dans lequel au moins une partie des éléments de massage (50) sont réalisés ou prévus sur le corps de base (51) sous la forme d'élévations en forme de nervures ou de vagues.

5. Bandage pour genou selon l'une des revendications 2 à 4, dans lequel les éléments de massage (50) présentent des dimensions comprises entre 1 mm et 8 mm perpendiculairement ou dans une direction opposée au corps de base (51).

6. Bandage pour genou selon l'une des revendications précédentes, dans lequel la pelote de massage (5) ou les éléments de massage (50) sont réalisés dans un matériau élastomère, en particulier un élastomère à base de silicone ou à base de polysiloxalane, et présentent de préférence une dureté Shore A comprise entre 20 et 80 Shore A, en particulier entre 55 et 65 Shore A.

7. Bandage pour genou selon l'une des revendications précédentes, dans lequel la pelote de massage (5) est agencée dans une poche (25) sur le côté intérieur tourné vers la jambe de l'élément de base du bandage (2).

8. Bandage pour genou selon l'une des revendications précédentes comprenant au moins un élément de correction (42), en particulier une bande de correction ou un arceau de correction, qui est agencé sur l'élément de base du bandage (2) sur un côté latéral de la rotule afin d'exercer une force latérale sur la rotule de façon médiane vers l'intérieur.

9. Bandage pour genou selon l'une des revendications précédentes, dans lequel l'élément de serrage comprend une sangle de serrage (6) qui s'étend en oblique au-dessus de la zone pour cuisse (21) de l'élément de base du bandage (2) jusqu'à la zone de transition vers la zone pour rotule (20) ou jusqu'à la zone pour rotule et/ou qui est réglable en longueur afin de modifier la force de serrage.

10. Bandage pour genou selon la revendication 9, dans lequel la sangle de serrage (6) présente deux parties de sangle de serrage (60, 62) qui sont chacune fixées, en particulier collées ou cousues, dans leurs zones d'extrémité situées en opposition l'une avec l'autre sur l'élément de base du bandage (2), en particulier sur sa surface extérieure opposée à la jambe, et qui sont reliées ou peuvent être reliées l'une avec l'autre dans leurs zones ou extrémités tournées l'une vers l'autre au moyen d'un élément de liaison, en particulier un oeillet (61).

11. Bandage pour genou selon la revendication 9 ou la revendication 10, dans lequel il est prévu sur l'une des parties de la sangle de serrage (62) des éléments de griffe au moyen desquels la longueur de cette partie de la sangle de serrage (62) peut être réglée.

12. Bandage pour genou selon l'une des revendications précédentes, dans lequel l'élément de serrage comprend au moins un élément intermédiaire, en particulier élastique, qui est calé ou peut être calé ou qui est agencé ou peut être agencé entre, d'une part, une sangle de serrage ou bande de serrage ou une zone élastique de l'élément de base du bandage, et d'autre part, la zone de massage comprenant la pelote de massage et qui exerce ainsi une pression sur la zone de massage.

13. Bandage pour genou selon la revendication 12, dans lequel la pression d'application peut être réglée au moyen de l'épaisseur de l'élément intermédiaire, et le cas échéant, de son élasticité et/ou dans lequel au moins deux éléments intermédiaires sont inclus, qui peuvent être remplacés l'un par l'autre et qui présentent des épaisseurs et/ou des élasticités différentes.

14. Bandage pour genou selon l'une des revendications précédentes, dans lequel la zone de massage comprenant la pelote de massage est affectée au point ou à la zone de déclenchement dans la zone marginale distale ou la zone de transition du Musculus vastus lateralis qui s'étend latéralement vers ou dans le Retinaculum patellae.
